# EUROPEAN PATENT SPECIFICATION

(11) **EP 2 564 881 B1**
(45) Date of publication and mention of the grant of the patent: **17.08.2022**
(21) Application number: 10838439.7
(22) Date of filing: 19.11.2010
(51) Int. Cl.: A61L 27/46, A61L 27/58, A61F 2/00, A61L 31/12, A61L 31/14

(54) **IMPLANTABLE MEDICAL OR VETERINARY DEVICE AND USE THEREOF**
IMPLANTIERBARE MEDIZINISCHE ODER VETERINÄRE VORRICHTUNG UND DEREN VERWENDUNG
DISPOSITIF MÉDICAL OU VÉTÉRINAIRE ET SON UTILISATION

(30) Priority: 22.12.2009 BR PI0906274
(43) Date of publication of application: 06.03.2013
(73) Proprietor: Wykrota, Francisco Henrique Lanna, 34000-000 Nova Lima - MG (BR)
(72) Inventor: Wykrota, Francisco Henrique Lanna, 34000-000 Nova Lima - MG (BR)
(74) Representative: Plasseraud IP
(86) International application number: PCT/BR2010/000391
(87) International publication number: WO 2011/075803

(56) References cited:
- WO-A1-2007/125323
- WO-A1-2007/134419
- WO-A1-2010/141718
- WO-A2-2008/100534
- BR-A- 9 104 220
- BR-A- PI0 602 372
- US-A1- 2002 115 742
- US-A1- 2002 115 742
- US-A1- 2006 136 068

## Description

The present patent relates to and concerns medical and veterinary devices intended for being implanted and transplanted into both humans and vertebrate animals, aimed mainly at restoring, reconstructing, remodeling, and performing plastic surgeries of muscle and skeletal structures, with said devices becoming an integral part of the system, with no need of being removed.

This patent concerns a new state of the art of the development described in patent BR PI9104220-8, based on the use of new products and composites, and systems as per the **Bone Morphogenetic Protein,** used mainly in repairing the muscle and skeleton system, and also of the development laid down in patent file BR PI06022372-0, and a new development of BR PI9104220-8.

The technological knowledge that govern physiology mechanisms, biocompatibility, adherence, regeneration, tissue reconstruction, deposition, and desirable tissue differentiation have made it possible to develop these new implantable devices.

To meet this goal, nanostructured phosphocalcic material is used as per BR PI9104220-8 "PROCESS FOR OBTAINING BIOCERAMIC MATERIAL AND IMPLANT OF BONE MORPHOGENETIC PROTEIN", obtaining composites, BR PI0602372-0, "PROCESS FOR PREPARING COMPOSITES BASED ON BIOCERAMIC AND BIODEGRADABLE POLYMERS, CONTAINING ANTIBIOTICS AND ANGIOTENSIN(1-7), WHETHER OR NOT INCAPSULATED, MICRO- OR NANOPARTICULATED, AIMED AT RESTORING TISSUES OF DERIVATIVE PRODUCTS", by producing devices from whether or not biocompatible, biointegratable, biodegradable products.

Another characteristic of the invention is the potential use of substances that have been absorbed, conducted, and released in such concentrations that may vary pursuant to the indication, with desirable effects, for instance, anti-inflammatory, or tissue induction, such as osteogenic, in addition to having better biocompatibility and/or metabolic, curing, and cellular activity for the intended use.

Medical devices are produced by preparing composites based on materials obtained from calcium salts, mainly ceramic, that are associated and mixed with polymer materials, including zirconia polymer resin (ZRO₂).

These products are used for reconstructing tissues, and are obtained by the process involving the preparation of composites based on bioceramic materials, and are absorbed from the body and/or deposited, as well as polymers containing substances, whether or not encapsulated, micro or nanoparticulates, are further used as a matrix for support, proliferation, differentiation and endogenous and/or exogenous cellular culture.

The present patent concerns medical devices that, being biointegratable by interfaces, aim at reconstructing, remodeling or biologically, physiologically, aesthetically and/or anatomically filling certain structures of the human body and/or vertebrate animals.

### Comments on the State of the Art

The State of the Art describes a number of devices used for such purposes with only a few of them presenting the biointegration property, mostly partially, whilst the present devices favor total interface biointegration and conduction, in addition to the property of induction and support for tissue neoformation, becoming an integral part of the repairing, curing and scaring process.

Other problems posed by current devices concern their temporary permanence, without being replaced by an organic structure and/or with no desirable biological and physiological biointegration, nor any appropriate remodeling of the organic structure, structures that should be removed or are not biologically biolintegratable.

The devices made available by currently being used in connection with the State of the Art, such as rods, plates, pins, bolts, membranes, cages (intersomatic devices), cranial and facial bone overlapping, and cranioplasties, pose problems arising out of the elasticity module if compared with those of the natural tissues, mainly the bone tissue, as the interface is not fully biointegrated and the reabsorbtion cannot be anticipated, thereby contributing to excessive stress, in addition to the mold not being customized, except for a few special cases, which adds to the transmission of undesirable efforts as these are not part of the organic or the anatomic structure.

### Summary of the invention

The invention relates to an implantable medical or veterinary device, obtained from a process that comprises mixing particles of a nanostructured phosphocalcic material ranging from 4000 to 37 micrometres (5 to 400 mesh) with one of: a) PLA, PLGA or caprolactone; b) PMMA; c) a moldable phosphocalcic cementing substance; and sintering the resulting mixture to obtain a composite mineral nanostructured matrix making the mixture fully ceramized; characterized by comprising a matrix resulting from sintering of: a) a mixture of particles of a nanostructured phosphocalcic material ranging from 4000 to 37 micrometres (5 to 400 mesh) and a biodegradable polymer chosen from PLA, PLGA or caprolactone; b) a mixture of particles of a nanostructured phosphocalcic material ranging from 4000 to 37 micrometres (5 to 400 mesh) and PMMA; and c) further comprising absorbed substance which are conductible and releasable during use, preferably the absorbed substance is chosen from proteins, progenitor cells, endogenous cells and/or exogenous cells. The invention further relates to the use of said implantable medical or veterinary device as a matrix for fabricating an intramedular rod, an intersomatic cage device, a craniofacial anatomic and aesthetic reconstruction device, a device for suprabone craniofacial reconstruction plasties, a device for bone structural reconstruction, including osteoarticular, partial, total and implants and endo-implants, a membrane, a plate or a fastening bolt.

### Advancements introduced by this patent to the State of the Art

Owing to the difference between available conventional models, mainly due to the fact that there were no nanostructured materials in their composition, the aforementioned devices were developed as the object of this patent based on the knowledge that it was necessary to have products that could be fully incorporated by the organism of which such devices become an integral part, without any need of being removed, as they participate bioactively in the process.

These devices were manufactured based on such composites and, therefore, their properties lead to the induction and conduction of repairing tissues as they bear a mineral nanostructured matrix whose geometry favors the deposition of the cellular matrix, mineral deposition, and mineralization when, preferably, next to bone tissues, whose features and properties surpass those of the current State of the Art devices.

With such compositions, features and properties, the devices of the present invention lead to a more effective repair and/or formation of the tissue, showing a histological response that is compatible with the ordinary tissue structure, therefore, being capable, for instance, of withstanding the osteogenesis, in case of being used on bone tissues, or the chondrogenesis, if used on cartilaginous tissues to repair articulation structures, for instance.

Whether or not hydrolysable biodegradable polymers and/or cementing substances such as phosphocalcic products are used in the composition, specific devices can be manufactured and modeled in a customized way, using a more suitable elasticity module, with full and perfect biointegration by the interface, and a porosity that allows for the organic fluids flow, thereby making for a local personal self-defense mechanism and also the deposition of substances and organic structures that belong to the person and that are required for the repairing process and/or neoformation by chemotaxis and haptotaxis, and/or the required tissue juxtaposition, in compliance with the bone morphogenetic protein, which represent the first principles that characterize the present invention and an evolution of the state of the art, as a new development that creates a local condition for such and including:
- Composites of nanostructured phosphocalcic materials (e.g., OSTEOSYNT^{®}), and biodegradable polymers, selected from PLA, PLGA, and CAPROLACTONE, in different molar proportions, whose characteristics may be hot- or cold-molded. Components proportions may range from 10% to 90%, depending on the needs and indications, although preferably with molar proportions ranging from 50% to 75%, for OSTEOSYNT^{®}, and 50% to 25%, reabsorbable polymer substances.
- Composites of nanostructured phosphocalcic materials (e.g., OSTEOSYNT^{®}), and the non-resorbable polymer PMMA, in different molar proportions, whose characteristics may be cold- or hot-molded. Components proportions may vary pursuant to the needs, ranging from 10% to 90%, although preferably in the molar proportions of 60% to 90%, for OSTEOSYNT^{®} and 40% to 10%, for PMMA.
- Composites of nanostructured phosphocalcic materials, OSTEOSYNT^{®}, and phosphocalcic cementing different substances in different molar proportions, with moldable characteristics. Components proportions may vary from 10% to 90%, pursuant to the needs, although preferably in the molar proportions between 70% and 90% of OSTEOSYNT^{®}, and 30% and 10% of phosphocalcic cementing substances.

So, the new conception as developed by these new devices and system, with due consideration being given to the composition's possible variations, can be better evaluated by looking at the attached drawings.

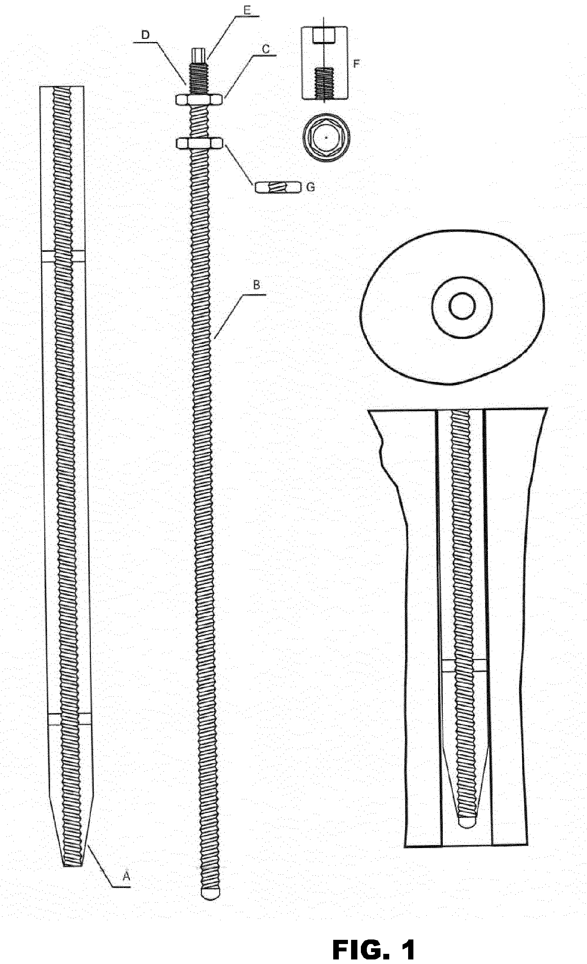

### FIG 1 - Shows an exploded view of the intramedullary rod device constituent elements.

**A** - The rod itself.
**B** - Internal device made of steel or carbon fiber to serve as a guide, removable, threaded or not (either right-handed or left-handed), with blunt point and a preferably trapezoidal upper end, although other forms are allowable, for the absorption and distribution of impact and introduction forces.
**C** - B device backrest for F device, at the upper portion and for the rod's lower portion.
**D** - Part of B device with left-handed (or right-handed) thread for the F device to fit in.
**E** - Hex head for the introduction and/or removal (optional) of B device.
**F** - B device upper end to withstand impact, and internal left-headed (or right-headed) thread for introduction and use, and for the removal of said B device.
**G** - Hex head washer, internal thread for adjusting the backrest height on B device.

This medical-veterinary device, herein referred to as an intramedullary rod, is used to fasten long bones, preferably in the event of fractures and/or when there is no consolidation of greater extension and seriousness, for instance, which, upon being inserted into the medullary channel, favors bone structure realignment, focus compression and fastening, faster stability, curing recuperation, and movement of limbs and other structures.

In addition to being highly rigid and having no flexibility, current available steel devices are rather traumatic upon insertion, after the medullary channel is milled/drilled. These devices are fastened to proximal and distal ends, following their compression from the ends, provides stability from the two fastening points only, as there is no total interfaces biointegration, nor partial integration between devices and the organic tissue.

Following consolidation and cure, this device must be removed, mainly because a traumatic impact could lead to serious injuries brought about by the very itself.

Other important aspects regarding current devices is the lack of metabolic/cellular activity within the space occupied by them, which compromises the organic/self defense system, in addition to the reduction of organic fluids flow. This may further lead to a never-ending inflammatory process caused by the contact with non-biodegradable surfaces, due to the exfoliation brought about by the person's physical activities and his/her reflexes, by the difference of elasticity modules between bone tissue and these structures (e.g., metallic).

In addition to eliminating these disadvantages, the aforementioned new state of the art device induces metabolic and cell activity, as it has in its composition, and mainly on its surface, great volume of bioceramic and bioactive material mainly for the osteogenesis.

It allows the relative flow of organic fluids, thereby increasing self organic defense, due to greater or smaller permeability, has a compatible elasticity model and, in addition to being fully biointegratable all over its structure, it is reabsorbable over time, as is the case of the first fastening devices for the ends, which work as a single body together with adjacent tissues, thereby eliminating the need for a removal procedure.

Its gradual absorption allows for a desirable bone reconstruction and remodeling. It can further be used as a media for the conduction of such substances as antibiotics, angiotensin, other proteins, and bone morphogenetic proteins (BMPs). Additionally, it is used as an endogenous and exogenous culture media, and as a matrix, it may also support the process with the insertion of culture cells. It may feature different dimensions, length, both external and internal diameter, and the same proportional variation of the insertion device, depending on the use.

Its structure allows for the insertion of the internal steel device in order to facilitate its insertion and maintain the integrity of such procedures. Whether or not it is immediately removed, it favors the maintenance of an intramedullary channel, aimed at desirable metabolic activities and/or an exogenous introduction of substances and structures, such as antibiotics and cells cultivated as therapy. Owing to the superficial treatment that exposes the phosphocalcic material, it leads to a fast total biointegration. Another advantage is that the device can be manufactured in a customized way, based on 3D modeling, which allows it to fit perfectly into the implanted patient's organic structure, thereby facilitating the procedure and reducing surgery and recovery time.

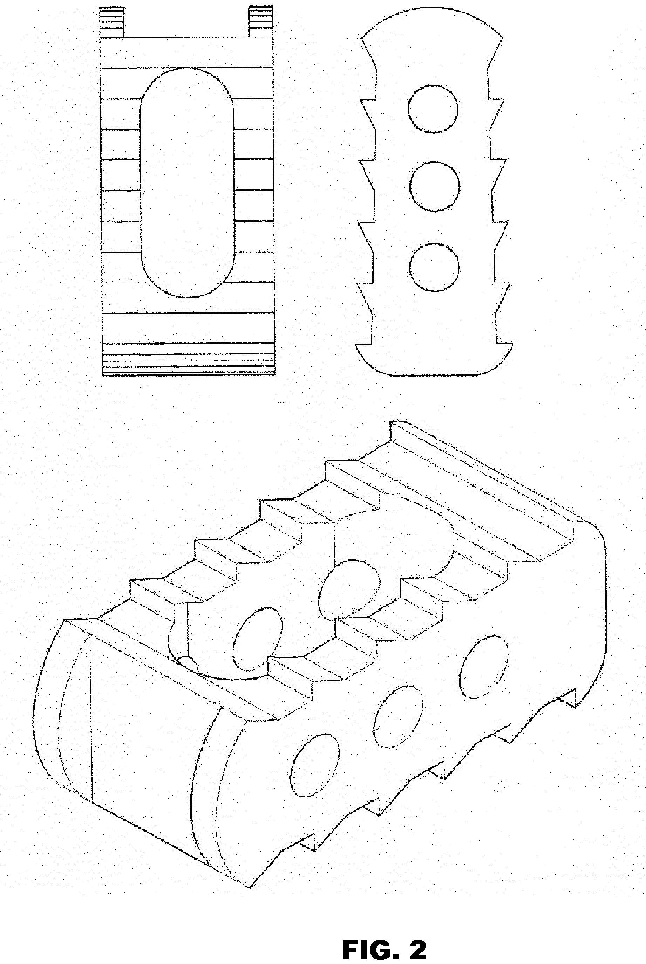

### FIG 2 - Intersomatic cage device

As previously described, the intersomatic cage device is largely used in processes in which the maintenance of the anatomic dimension of intervertebral spaces so as to avoid the compression of nerve roots and, mainly, structural deformities. Currently, these devices are made available mostly in steel and/or polymer non-biodegradable products, which almost always requires the use of alternative bone graft material or associated autologous bone graft to fill internal spaces left by these devices, in addition to external gaps between vertebrae aimed at consolidation and arthrodesis, if any, requiring strong and extensive external steel fastening.

In addition to the appropriate mechanical resistance provided by the composite, the new devices of the aforementioned state of the art involving phosphocalcic materials (**OSTEOSYNT**^{®}), which have been described in patent PI 9104220-8, and non-absorbable polymer, lead to total biointegration of the interfaces device, namely, between the latter and vertebral bone surfaces, in addition to a complete arthrodesis and, in many cases, without the need of an external metallic fastening that, if performed, becomes dispensable over time.

The intersomatic device allows for the flow of organic fluids and cellular activity, thereby speeding up the curing process and in addition to being a matrix for cellular culture they may also be used as a vehicle for the conduction and release of drugs and substances.

Another advantage is that the device can be manufactured in a customized way, through 3D modeling, in a way to suit the implanted person's anatomy, facilitating the execution of the procedure and ensuring better results in less time. This device may also be manufactured in diverse manners of format and presentation, whether indicated or required, and in various dimensions for the required use.

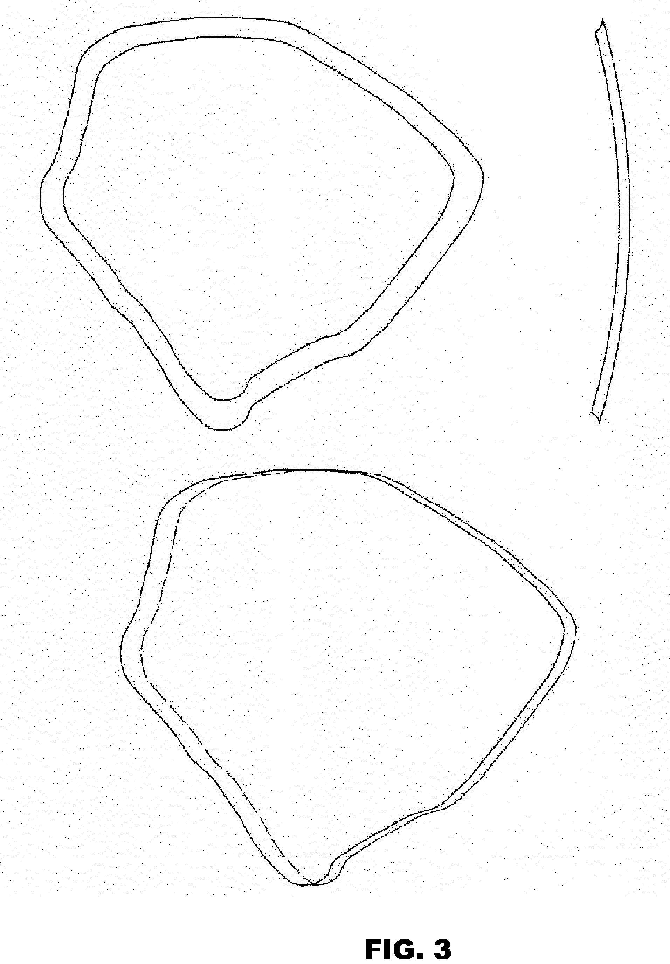

### FIG 3 - Craniofacial anatomic and aesthetic reconstruction devices

This figure depicts the specific parts for reconstruction craniofacial plasties.

These parts must be manufactured with composites based on nanostructured calcium salts materials, such as HA/TCP OSTENSYNT^{®} (PI 9104220-8) biphasic, micro-macro, porous bioceramics associated to reabsorbable polymers, or not, and/or phosphocalcic cementing substances, depending on the use indication and the patient's pathology under treatment.

Whenever an immediate anatomic and/or aesthetic reconstruction is needed, such as the mechanical protection of organs and structures, as well as in the case of oncologic patients, in which case there is the risk of recurrence due to cellular metabolic activity, devices obtained with composites containing nondegradable polymers are most indicated. The initial and superficial biointegration allows for immediate adherence to the adjacent tissue, thereby becoming a single body that is capable of withstanding mechanical efforts and the natural distribution of force vectors, as a remnant of the constructed structure.

On the other hand, they can also be manufactured with composites containing reabsorbable polymers or compounds with phosphocalcic cementing substances, and can further be totally ceramized, whereby all become an integral part of the reconstructed structured in a quicker way.

Furthermore, they be provided with more rigid and resistant external layers, with a more porous internal layer, under several presentation formats and models, whether ordinary, as per indication and use, and generally produced based on 3D digital images of the patient to suit the indicated use.

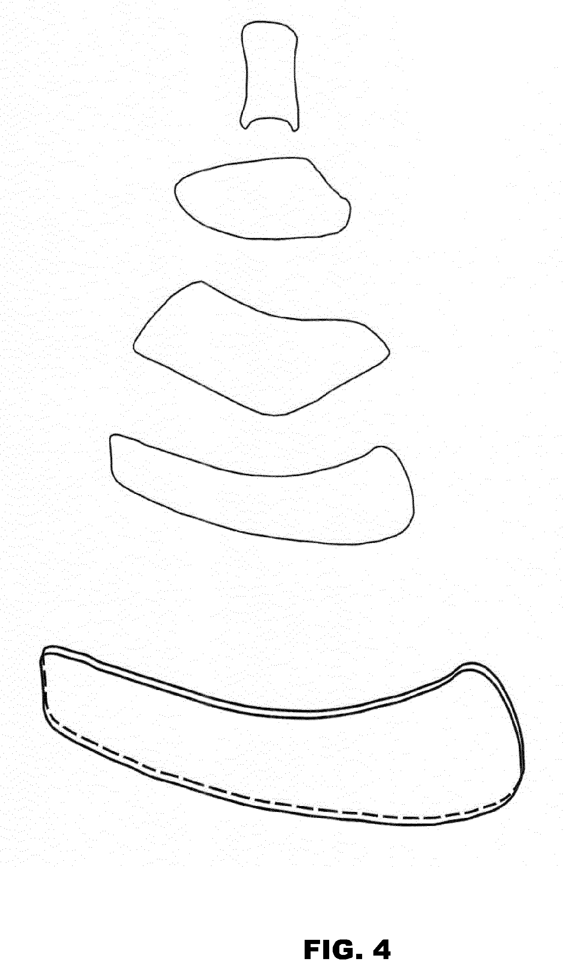

### FIG 4 - Device for suprabone craniofacial reconstruction plasties

The described devices aimed at aesthetic, anatomic, suprabone, and plasties (e.g., craniofacial) are preferably manufactures from composites, with nanostructured, phosphocalcic materials, NA/TCP biphasic micro-macro porous ceramics (OSTEOSYNT^{®}), BR PI9104220-8, associated to reabsorbable, moldable, and flexible polymers, such as caprolactone, mainly, PLA, PLG, PLGA, thereby allowing for perfect handling and fastening with mechanical resistance. Upon being applied to previously prepared bone surfaces, these devices are quickly biointegrated, which allows for the neoformation of desired tissue, instead of undesirable ones like fibrotic tissues. The devices then become part of the tissue's adjacent structural part as opposed to currently available devices that are made of non-degradable polymers such as linear high density polyethylene and non-biodegradable interfaces, with no desirable bioactivity.

These devices may also be made in a customized way using digital 3D drawings of the individual, in diverse format and dimensions to suit the indicated use. Furthermore, in addition to being able to release substances, they are cellular culture matrixes and, thereby, facilitate the procedure, reduce both surgery and recovery time, and do not lead to undesirable reactions.

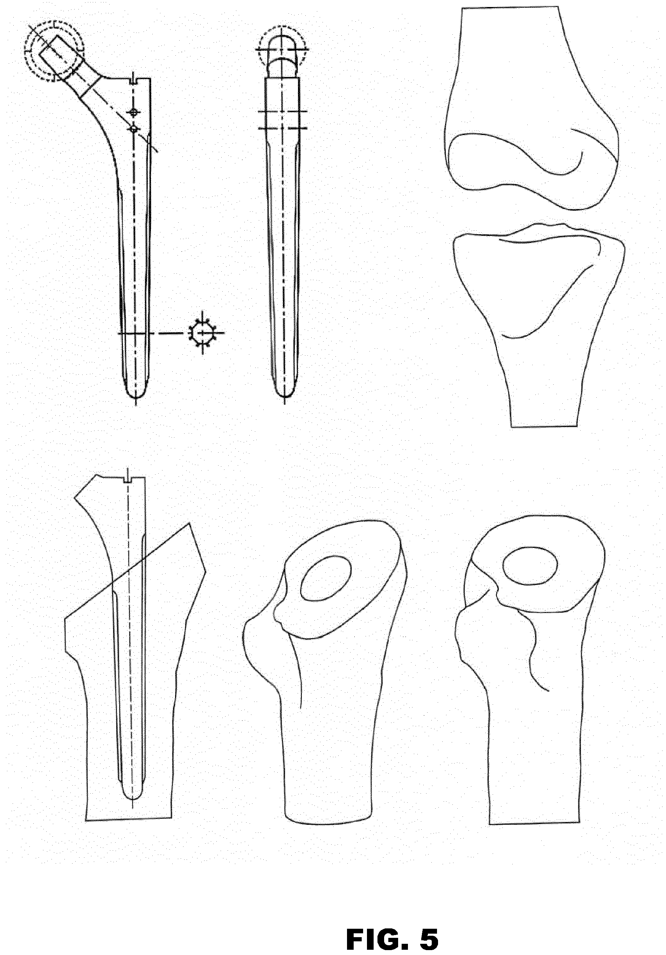

### FIG 5 - Devices for bone structural reconstruction, including osteoarticular, partial, total and implants and endo-implants.

The devices shown in figure 5 are used for total and/or partial reconstruction of bone structures and/or articular cartilagenous bone structures, as well as for plasties involving implants and endoprosthesis.

These shall be produced and applied using composites from nanostructured phosphocalcic, HA/TCP (OSTEOSYNT^{®}), BR PI9104220-8 biphasic micro-macro porous bioceramics, associated to biodegradable (or not) polymers, and phosphocalcic cementing substances, fully ceramized, depending on the indication and use, in cases in which an immediate aesthetic reconstruction is needed along with muscle and soft tissues adherence. The composite may be used as a non-reabsorbable polymer to coat implants and endoimplants, or to reconstruct such areas subject to efforts as the acetabular cavity.

These may be produced with reabsorbable polymers and/or phosphocalcic substances to support cellular culture and exogenous tissue genesis to be later implanted without making use of any cellular culture, being mainly transplanted in the case of bone cartilaginous articular reconstructions, which allowing for the structure's natural reconstruction, which so far have not been made available in the market, thereby allowing for the replacement of other metal devices. These new devices are generally specific parts produced via 3D modeling of the individual's image pursuant to his/her own anatomic and aesthetic characteristics based on indication and use.

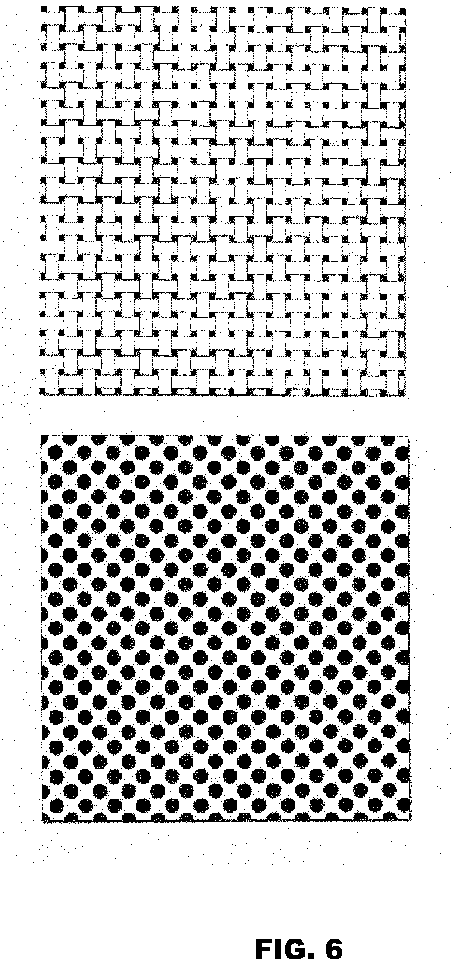

### FIG 6 - Membranes

The devices shown in figure 6 are containment and/or guided regeneration membranes. These devices are produced with nanostructured phosphocalcic materials, HA/TCP (OSTEOSYNT^{®}), BR PI9104220-8 biphasic micro-macro porous bioceramics, in composites with reabsorbable polymers, PLA, PLG, PLGA, (e.g., caprolactone), mainly, or sol/gel.

These devices have variable permeability along with a protection and containment barrier. As a cellular culture matrix, they serve as a guide to the tissue neoformation, and may drag and release substances and organic structures, and/or those needed in the process.

These devices are preferably moldable with mechanical resistance for traction and suture, with flexibility, depending on the composition.

As they come in different compositions, they facilitate their use and indication, in addition to being a matrix for both chondrogenesis and osteogenesis, and may additionally be used for recovering epithelial tissues in cases involving burns, but may also be meshes made from composites filaments.

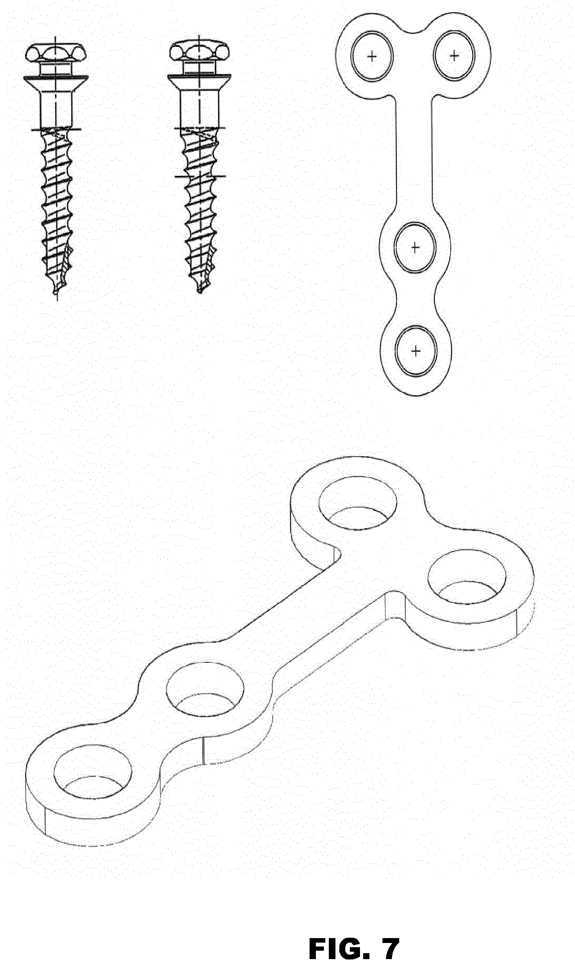

### FIG 7 - Plates and fastening bolts

These devices may be produced via pressing, compression, extrusion and/or injection of moldable composites, with execution forces being varied in order to define greater or smaller porosity, and/or greater or smaller mechanical resistance, as well as temperature variations during manufacturing, as it happens with all previously described devices.

Nanostructured phosphocalcic materials such as the biphasic micro-macro porous ceramics (**OSTEOSYNT**^{®}), as per BR PI9104220-8, typify the composites crucial phase by the excellent conducting and inducting capacity through own chemotaxis and haptotaxis. Moreover, it features the capacity of absorbing, conducing and releasing substances through superficial stress arising out of its physical and architectural structure, it form and intercommunicating porosity, synergized by the chemical composition that may contain variable rates for CA/P, thereby influencing greater or smaller reabsorption ratios, particle size with mechanical resistance and appropriate physicochemical properties.

These structures also allow for the continuous flow of fluids and carried organic substances, and their deposition onto this matrix, for instance, as is the case of blood and hemoderivatives, as is the case of all described devices.

Reabsorbable polymeric materials mixed pursuant to the ratio of applied percentage and their characteristic reabsorption rate allow for and lead to manufacturing devices with elasticity module that suit the indicated use, in addition to a most ideal reabsorption rate for the indication. Their gradual reabsorption exposes the phosphocalcic material, and as a result, in a gradual and progressing manner, which, upon being associated to compatible organic processes, leads to a desirable result, as is the case of intramedullary rods, plates, bolts, membranes, for instance, aimed at a better and faster tissue neoformation and ultimate replacement.

Non-absorbable polymeric materials that are mixed as per the applied percentage ratio allow for and lead to the manufacture of more stable, more rigid and everlasting devices, including the elasticity module, which are deemed necessary to the use with integrity, although showing fully integrated into the adjacent structure and totally and desirably biointegrated, mainly the immediate superficial structure, as it happens with all devices.

Phosphocalcic cementing materials that have been mixed as per the required and indicated rate, allows for manufacturing devices with greater relative resistance to the elasticity module compatible with the use, and gradual absorption up to complete absorption by an organic tissue, making for good porosity capacity and fluidity of organic liquids, and may also be fully ceramized as needed.

Mixing these substances and/or whatever products are available in the market, as indicated in the presentation form, allow for the combination of each one's advantages with synergy, streamlining the process and reducing potential undesirable reactions to normal and organically bearable levels or else their elimination, with no aggravating inflammatory processes, but only biologic, over each processes' due time, thereby speeding up the process. This is also due to the inexistence of organic substances that can be potentially reabsorbed by necrosis, for instance, as is the case of substances that do not favor reabsorption due to lack of vascularization that characterizes autogenous grafts, and even homologous and xenologous grafts, or any other inserted exogenous substance as it happens in all situations in which the use of such devices is indicated. Accordingly, when specifically indicated reabsorbable polymers are used in the devices, object of the present invention, and due to the effort sealing, no bony substance is lost with the implanted support metal, with the curing process being intensified as mainly the bone gradually takes on the responsibility for the mechanical effort over the degradation process and/or replacement of the graft, thereby speeding up the curing process through the physiological feedback mechanism, everything happening at the same time. Other advantages, such as identifying them, elimination of reflection and distortion of radiologic images, help analyze the curing process by its composition.

In addition to medical advantages, economical and risk-related reasons recommend the use of the devices, object of the present patent application, considering that there is no cost involved with operating on patients, mainly for the removal metal devices, thereby eliminating not only the risk associated with other surgeries and the shut-in time thereof. These composites are provenly capable of withstanding both the osteogenesis and the chondrogenesis process.

The degradation of these reabsorbable polymer materials takes place when they react with organic fluids to decompose its molecular chain at which point the ressorption and metabolic elimination take place.

The stability and rigidity of these materials is rather encompassing, ranging from 30 to 100 Mpa and 2000 to 5000 Mpa, respectively, allowing for a broad range of use as per indication, in addition to a reabsorption period that may vary from 2 to 24 months. These substances are considered in ASTM - D-883-99 standards. On the other hand, devices made by mixing non-degradable

PMMA, with the mineral matrix (**OSTEOSYNT**^{®}) allows for an increased level of resilience and stability, thereby enabling them to absorb expressive impacts and efforts, mainly compression, due to the use of compatible elasticity modules. Furthermore, these devices can also work nicely as protection mechanisms for organic structures.

The appropriate viscosity to be used as the required treatment developed for the removal of residual monomers for the general production of the aforementioned products, so that they can be produced in an atoxic manner, is considered in these composites.

This device is implanted with fluids at a body temperature of 37°C, and being saturated with these fluids, the elasticity module fits in and, as a result, there is a desirable mechanical behavior, thereby improving the composite's physical features. These polymers (e.g., PMMA) have been used for over fifty years in both medical and dentistry procedures, and with the technique developed for the new composite, considering the indicated mineral matrix (**OSTEOSYNT**^{®}), it shows an outstanding adhesion, proliferation and cellular differentiation capacity, in addition to total biocompatibility and osteointegration. Therefore, in addition to presenting no undesirable reaction, PMMA also provides significant advantages when used in permanent devices aimed mainly at plastic reconstructions, flat bone aesthetics, and maintenance of anatomic spaces or as a guide for tissue formation in long bones. It can also be used to replace small bone structures like hands and feet, or even in the case of longer and bigger bones.

Mixing the mineral matrix (**OSTEOSYNT**^{®}) with phosphocalcic cementing substances leads to the manufacture of more rigid and mechanically resistant devices, capable of absorbing body liquids and substances, with subsequent adequacy to elasticity modules. With an appropriate reabsorption, it can be used in plastic surgeries, mainly those involving bones in general, in addition to being capable of withstanding, for example, osteogenesis and chondrogenesis, with all characteristics and properties of the association of phosphocalcic materials being kept in synergy. Additionally, it shows a high level capacity of adhesion, multiplication and faster cellular differentiation, and subsequent repair and tissue remodeling.

All devices may be individually and specifically produced in a customized way, using 3D digital modeling based on radiological images generated by a process developed by EINCO BIOMATERIAL or in a series, in various desirable formats.

All devices undergo treatment of their surfaces aimed to secure an ideal topography, in order ensure an even more bioactive interface and, therefore, better biointegration.

These devices are produced based on a sintering process, after mixing components and making them fully ceramized, pursuant to the needed use and indication, in order to obtain biologically desirable physical and/or architectural structures, in the presence of only phosphocalcic materials, as per indicated.

All devices show an appropriate condition for the culture of osteoblasts based on a quantitative measurement of bone sialoprotein and osteopontin.

In addition to being obtained through the various relation ratios of these materials and polymer substances, all devices are produced by mixing diverse sizes of particles of nanostructured phosphocalcic material, ranging from 5 to 400 mesh, preferably for determining the desirable structure relative to elasticity module, mechanical resistance, porosity, fluid absorption rate, stability, flexibility, dimensions, reabsorption capacity, transport, substances release and body fluids flow and their content (e.g., blood), favoring a number of presentation and use formats, and, consequently, a desirable biological an physiological prosthetic articular reconstruction, inclusive.

These devices provide several advantages as opposed to the current state of the art, such as total biocompatibility, total biointegration, total biological and physiological interaction, total architectural, physical and structural adaptation, allowing for the dissipation of actuating forces, reduced surgery and recovery time, elimination of surgeries and the results associated with the removal of surgery-requiring devices, better cure, improved scaring along with a natural tissue remodeling, and restoration of both biological and physiological activities. These devices may be produced in various formats and structures, such as 3D modeling, specific and customized, in addition to being used as a matrix for cellular culture and differentiation, implantable and/or transplantable endogenous and exogenous reconstruction of organic structures due to their added capacity to absorb, conduct and release substances and required endogenous and exogenous structures, all of which being part of the processes.

## Claims

1. Implantable medical or veterinary device, obtained from a process that comprises mixing particles of a nanostructured phosphocalcic material ranging from 4000 to 37 micrometres (5 to 400 mesh) with one of:
a) PLA, PLGA or caprolactone;
b) PMMA; or
c) a moldable phosphocalcic cementing substance; and sintering the resulting mixture to obtain a composite mineral nanostructured matrix making the mixture fully ceramized;
**characterized by** comprising a matrix resulting from sintering of:
a) a mixture of particles of a nanostructured phosphocalcic material ranging from 4000 to 37 micrometres (5 to 400 mesh) and a biodegradable polymer chosen from PLA, PLGA or caprolactone;
b) a mixture of particles of a nanostructured phosphocalcic material ranging from 4000 to 37 micrometres (5 to 400 mesh) and PMMA; and
c) further comprising absorbed substance which are conductible and releasable during use, preferably the absorbed substance is chosen from proteins, progenitor cells, endogenous cells and/or exogenous cells.

2. The implantable medical or veterinary device according to claim 1, wherein in a), the biodegradable moldable polymer is chosen from reabsorbable PLA, PLGA or caprolactone.

3. The implantable medical or veterinary device according to the above claims, wherein the implantable medical or veterinary device is an intra-medular rod for fastening long bones.

4. The implantable medical or veterinary device according to claim 3, wherein the intra-medular rod comprises a longitudinal threaded cavity therethrough for the introduction of an internal device acting as guide.

5. The implantable medical or veterinary device according to one of claims 2 to 4, further comprising surfaces with compositions including sol/gel substances in the form of membranes and presenting architecture and topography allowing the full interfaces biointegration thereof.

6. Use of the implantable medical or veterinary device of claim 1 as a matrix for fabricating an intramedular rod, an intersomatic cage device, a craniofacial anatomic and aesthetic reconstruction device, a device for suprabone craniofacial reconstruction plasties, a device for bone structural reconstruction, including osteoarticular, partial, total and implants and endo-implants, a membrane, a plate or a fastening bolt.

7. The use of claim 6, wherein the implantable medical or veterinary device is used as a matrix of a culture membrane.

## Patentansprüche

1. Implantierbare medizinische oder veterinärmedizinische Vorrichtung, erhalten durch ein Verfahren, welches das Mischen von Teilchen eines nanostrukturierten phosphokalzischen Materials mit einer Größe von 4000 bis 37 Mikrometern (5 bis 400 mesh) mit einem der folgenden Materialien umfasst:
a) PLA, PLGA oder Caprolacton;
b) PMMA; oder
c) einer formbaren phosphokalzischen Zementsubstanz; und Sintern des resultierenden Gemischs, um eine mineralische nanostrukturierte Verbundmatrix zu erhalten, wodurch das Gemisch vollständig keramisiert wird;
**dadurch gekennzeichnet, dass** sie eine Matrix umfasst, die erhalten wird durch Sintern von:
a) einem Gemisch von Teilchen eines nanostrukturierten phosphokalzischen Materials mit einer Größe von 4000 bis 37 Mikrometern (5 bis 400 Mesh) und einem biologisch abbaubaren Polymer, das aus PLA, PLGA oder Caprolacton ausgewählt ist;
b) einem Gemisch von Teilchen eines nanostrukturierten phosphokalzischen Materials mit einer Größe von 4000 bis 37 Mikrometern (5 bis 400 Mesh) und PMMA; und
c) ferner eine absorbierte Substanz umfasst, die leitfähig und während der Verwendung freisetzbar ist, wobei die absorbierte Substanz vorzugsweise aus Proteinen, Vorläuferzellen, endogenen Zellen und/oder exogenen Zellen ausgewählt ist.

2. Implantierbare medizinische oder veterinärmedizinische Vorrichtung nach Anspruch 1, wobei in a) das biologisch abbaubare formbare Polymer aus resorbierbarem PLA, PLGA oder Caprolacton ausgewählt ist.

3. Implantierbare medizinische oder veterinärmedizinische Vorrichtung nach den vorhergehenden Ansprüchen, wobei die implantierbare medizinische oder veterinärmedizinische Vorrichtung ein intramedullärer Stab zur Befestigung langer Knochen ist.

4. Implantierbare medizinische oder veterinärmedizinische Vorrichtung nach Anspruch 3, wobei der intramedulläre Stab einen längs hindurch verlaufenden, mit einem Gewinde versehenen Hohlraum zum Einbringen einer internen Vorrichtung aufweist, die als Führung dient.

5. Implantierbare medizinische oder veterinärmedizinische Vorrichtung nach einem der Ansprüche 2 bis 4, welche ferner Oberflächen mit Zusammensetzungen umfasst, die Sol/Gel-Substanzen in Form von Membranen enthalten und eine Architektur und Topographie aufweisen, die eine vollständige Grenzflächen-Biointegration ermöglicht.

6. Verwendung der implantierbaren medizinischen oder veterinärmedizinischen Vorrichtung nach Anspruch 1 als Matrix für die Herstellung eines intramedullären Stabes, einer intersomatischen Cage-Vorrichtung, einer anatomischen und ästhetischen kraniofazialen Rekonstruktionsvorrichtung, einer Vorrichtung für kraniofaziale Suprabone-Rekonstruktionsplastiken, einer Vorrichtung für die strukturelle Rekonstruktion von Knochen, einschließlich osteoartikulärer, partieller und totaler Rekonstruktion sowie Implantaten und Endoimplantaten, einer Membran, einer Platte oder eines Befestigungsbolzens.

7. Verwendung nach Anspruch 6, wobei die implantierbare medizinische oder veterinärmedizinische Vorrichtung als Matrix einer Kulturmembran verwendet wird.

## Revendications

1. Dispositif médical ou vétérinaire implantable, obtenu à partir d'un procédé qui comprend le mélange de particules d'un matériau phosphocalcique nanostructuré allant de 4000 à 37 micromètres (5 à 400 mesh) avec l'un parmi :
a) du PLA, PLGA ou de la caprolactone ;
b) du PMMA; ou
c) une substance de cimentation phosphocalcique moulable ; et le frittage du mélange résultant pour obtenir une matrice nanostructurée minérale composite rendant le mélange entièrement céramisé ;
**caractérisé en ce qu'**il comprend une matrice résultant du frittage de :
a) un mélange de particules d'un matériau phosphocalcique nanostructuré allant de 4000 à 37 micromètres (5 à 400 mesh) et d'un polymère biodégradable choisi parmi le PLA, le PLGA ou la caprolactone ;
b) un mélange de particules d'un matériau phosphocalcique nanostructuré allant de 4000 à 37 micromètres (5 à 400 mesh) et de PMMA ; et
c) comprenant en outre une substance absorbée qui sont conductibles et libérables lors de l'utilisation, de préférence la substance absorbée est choisie parmi des protéines, des cellules progénitrices, des cellules endogènes et/ou des cellules exogènes.

2. Dispositif médical ou vétérinaire implantable selon la revendication 1, dans lequel en a), le polymère moulable biodégradable est choisi parmi le PLA, le PLGA ou la caprolactone réabsorbable.

3. Dispositif médical ou vétérinaire implantable selon les revendications ci-dessus, dans lequel le dispositif médical ou vétérinaire implantable est une tige intra-médullaire pour la fixation d'os longs.

4. Dispositif médical ou vétérinaire implantable selon la revendication 3, dans lequel la tige intra-médullaire comprend une cavité taraudée longitudinale la traversant pour l'introduction d'un dispositif interne formant guide.

5. Dispositif médical ou vétérinaire implantable selon l'une des revendications 2 à 4, comprenant en outre des surfaces avec des compositions comportant des substances sol/gel sous la forme de membranes et présentant une architecture et une topographie permettant leur bio-intégration complète aux interfaces.

6. Utilisation du dispositif médical ou vétérinaire implantable selon la revendication 1 en tant que matrice pour la fabrication d'une tige intramédullaire, d'un dispositif de cage intersomatique, d'un dispositif de reconstruction anatomique et esthétique cranio-faciale, d'un dispositif pour plasties supra-osseuses de reconstruction cranio-faciale, d'un dispositif pour reconstruction structurale osseuse, y compris ostéoarticulaire, partielle, totale et des implants et endo-implants, une membrane, une plaque ou un boulon de fixation.

7. Utilisation selon la revendication 6, dans laquelle le dispositif médical ou vétérinaire implantable est utilisé en tant que matrice d'une membrane de culture.
